**Europäisches Patentamt**

**European Patent Office**

**Office européen des brevets**

(19)

(11) Publication number: **0 045 668**

**A1**

# EUROPEAN PATENT APPLICATION

(12)

(21) Application number: **81303599.5**

(22) Date of filing: **06.08.81**

(51) Int. Cl.³: **A 61 M 5/14**
**F 16 K 7/08**

(30) Priority: **06.08.80 GB 8025633**

(43) Date of publication of application:
**10.02.82 Bulletin 82/6**

(84) Designated Contracting States:
**AT BE CH DE FR GB IT LI LU NL SE**

(71) Applicant: **Peter Steer Developments Limited**
**The Malt House Works, Station Road East Grinstead**
**Sussex RH19 1DJ(GB)**

(72) Inventor: **Steer, Peter Leslie**
**The Malt House Works Station Road**
**East Grinstead Sussex RH19 1DJ(GB)**

(74) Representative: **Fentiman, Denis Richard et al,**
**Elkington and Fife High Holborn House 52/54 High**
**Holborn**
**London WC1V 6SH(GB)**

(54) **Device for controlling the flow of liquid.**

(57) A flow control device primarily but not exclusively, for medical use, comprises a tube (1) through which fluid can flow. The tube is stretchable longitudinally of its axis. A core (4) which may be a metal rod, is located inside the tube. The outside of the core is spaced from the inside of the tube so as to define a passage through which fluid can flow. Stretcher members (2 and 3) are located on the outside of the tube and bear on flanged ends of the tube. Means are provided for forcing the stretcher members apart thereby to stretch the tube.

In a modification the core is made of stretchable material and the stretcher members are located on the core.

Fig .1

- 1 -

Title:       Device for controlling the flow of
             liquid

This invention relates to devices for controlling the flow of fluids through tubes.

An object of the invention is to provide a device which is particularly useful for controlling the flow rates of liquids in the medical field such as in drip-feed sets.

Devices for controlling the flow of liquids for intraveneous or enteric feeding or for blood administration or the like are well known in the medical field. Hitherto, all such controls have usually been based on the principle of decreasing the size of the passage inside a tube by the exertion of pressure on its outside wall, such a passage is known as a "lumen". For example, a catheter of rubber or rubber-like plastics material may have the flow of liquid through it controlled by means of a screw clamp or an eccentric cam or by roller mechanism. All such known devices have two major problems. The chief problem is that the tube, which is initially round, changes its cross-sectional shape and lumen area when it is subjected to a clamping action from the outisde. In order to obtain complete closure of a tube, very high pressures causing very high stress to the tube are required. Attempts have been made to overcome this difficulty, for example, by using crushing anvils or rollers which are shaped to fit round the outside of the tube. These attempts have only been partially successful. Another problem with known clamping devices is that because of the relatively high pressures exerted on the wall of the tube during the clamping action the "memory" of the material (i.e. the tendency of the material to resume its original shape) which is usually polyvinylchloride, becomes disorientated with the result that a control which is initially set by a medical attendant to give the required rate of flow becomes inaccurate due to the impaired "memory" of the material of the tube. This involves the necessity of continual checking of the flow control device by the attendant with frequent adjustment of the operation of the device.

The present invention seeks to avoid this disadvantage.

According to the invention, there is provided, a flow control device comprising a tube, a core arranged inside the tube, the outside of the core being spaced from the inside of the tube thereby to define a passage through which fluid can flow, at least one of the tube and core being longitudinally stretchable thereby to vary the size of the passage.

In one embodiment the device comprises a tube which is stretchable longitudinally of its axis, a core within the tube, the outside of the core being spaced from the inside of the tube when the tube is in its unstretchable condition, stretcher members spaced from each other on the tube or catheter and fixed thereon, and means for forcing apart the stretcher members thereby to stretch the tube. With this arrangement, stretching of the tube or catheter will cause the space between the outside of the core and the inside of the tube or catheter to be diminished thereby to reduce the size of the passage through which fluid can flow through the tube or catheter. In an extreme position, the inside of the tube or catheter will contact the outside of the core thereby completely closing the device against the flow of fluid therethrough. In the other extreme position, the tube or catheter is in the unstretched condition so that the space between the outside of the core and the inside of the tube or catheter is at its maximum, thereby to permit maximum flow of fluid through the device.

In a modification, the flow control device has a tube or catheter which is not made of stretchable material, but the core is made of such material and the stretcher members are anchored to the core.

In the accompanying drawings:

Figure 1 is a schematic sectional view, showing a flow control device according to this invention in its inoperative position permitting maximum flow,

Figure 2 is a similar view illustrating the same device in a stretched condition providing maximum flow of liquid through the device.

Figures 3 and 4 are sectional views illustrating one practical embodiment of the invention,

Figure 5 is an exploded perspective view of the same embodiment, and

Figures 6 and 7 are views similar to Figures 1 and 2 illustrating a modification.

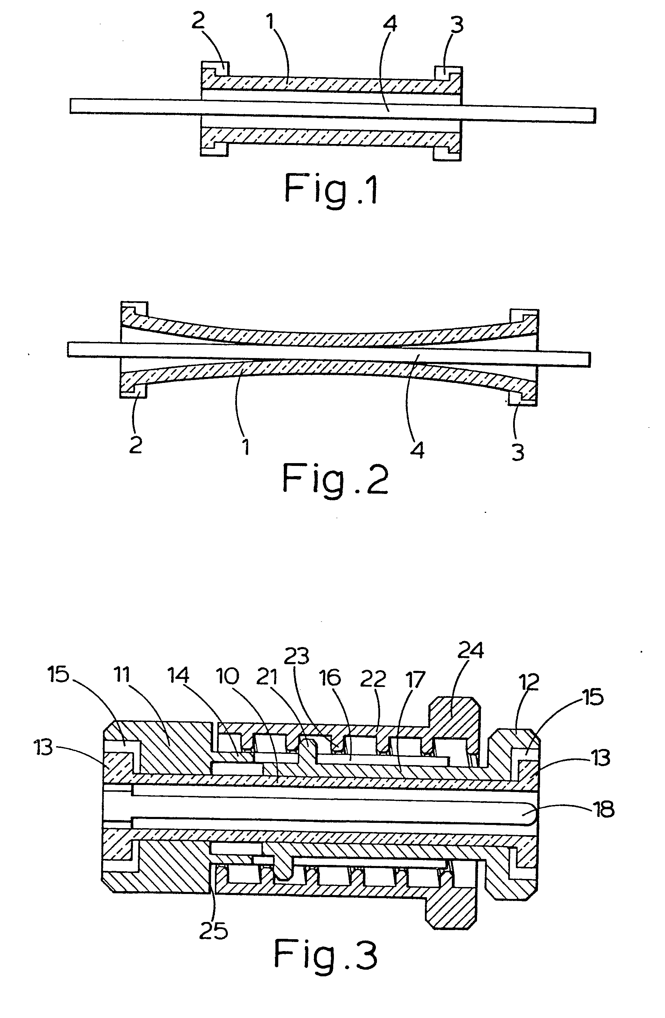

The basic principle underlying the invention is illustrated in Figures 1 and 2 of the drawings. As illustrated in these Figures, a flow control device comprises a tube or catheter 1 which is conveniently made of latex or silicon rubber or some other thermoplastic material which has good elastic properties and which will stretch with a decrease in diameter of the tube. Two bobbin members 2 and 3 are anchored to, or integral with, the tube or catheter 1 at spaced positions. Inside the tube or catheter is a core, which may be a metal rod 4, the diameter of the core being such that an annular space is left between the outside of the core and the interior of the lumen of the tube or catheter. Figure 1 shows the device in its normal or inoperative condition in which the annular passage between the core and the inside of the tube or catheter is at its maximum. In this position, the maximum amount of fluid can pass through the lumen of the tube or catheter. Figure 2 illustrates the same device when the bobbins 2 and 3 have been moved apart by a sufficient amount so to stretch the tube or catheter that the interior walls of the tube or catheter contact the exterior walls of the core. In this fully stretched condition the passage between the core and the inside of the tube or catheter is closed and no fluid can pass therethrough.

Figures 3 and 5 illustrate a practical embodiment making use of this principle to control the flow of liquid through a drip feedset for use in surgery. In this embodiment, a tube or catheter 10 which can be longitudinally stretched and which when stretched, reduces in internal diameter, has two flanged ends 13 at suitably spaced positions. Stretcher bobbins 11 and 12 about the flanged ends 13. The bobbin 11 is a female bobbin having a tubular extension 14 of narrower diameter. A passage extends through the female bobbin 11 so that the bobbin is an easy sliding fit on the outside of the tube or catheter. Collets 15 anchor the bobbin 11 to the tube or catheter. Alternatively, a hollow frusto-conical wedge member may be forced into an open end of the tube or catheter 10 so that the tube or catheter is wedged or anchored to the female bobbin 11. The extension 14 of the female bobbin has a pair of diametrically opposed slots 16 (Figure 5) for a purpose to be described. The other bobbin member 12 is a male member having a tubular extension 17 which is an easy sliding fit in the tubular extension 13 of the female bobbin member. A passage extends through the male bobbin member and is of such a diameter that this member is also an easy sliding fit on the outside of the tube or catheter 10. Further collets 15 secure the tube or catheter to the male bobbin 12.

A solid core 18 is fitted into the interior of the tube or catheter 10 through the open end of the tube or catheter adjacent the open end of the female bobbin.

This core is secured in the open end by means of a locating flange 19 or in any other way. For example, it may be secured by a frusto-conical wedge (not shown) which is fitted to the core and is jammed into the open end of the tube or catheter 10 thereby to anchor it to the bobbin member 12.

The flange 19 has ports 20 (Figure 5) which permit liquid to be introduced into the annular space between the core 18 and the interior of the tube or catheter 10.

At one end of the extension 17 of the male bobbin member 12 are diametrically opposed ribs or projections 21 which are shaped as portions of a screw thread. These portions engage in the slots 16 of the extension 13 of the female member and therefore prevent the male and female bobbin members being rotated with respect to each other. The projections 21 project beyond the external surface of the extension 13 of the female bobbin member. An internally screw threaded sleeve 22 is fitted on the outside of the extension 13 of the female spool member 11 and can rotate thereon and is also axially movable thereon. The internal screw thread 23 of the sleeve 22 meshes with the opposed projections 21 on the extension 17 of the male bobbin member 12. Thus, when the sleeve 22 is rotated it will also be displaced axially on the extension 13. The sleeve 22 can have an outwardly directed flange 24 with a knurled edge which makes it easier to rotate the sleeve. When the sleeve 23 has been sufficiently rotated the flange 24 will be brought into engagement with a shoulder 25 between the main portion of the female bobbin member 11 and its extension 13. Continued rotation of the sleeve 23 will therefore force the two bobbin members apart thereby to stretch the tube or catheter and reduce the internal diameter of the tube or catheter 10. Thus, the flow of liquid through the annular passage between the core 18 and the inside of the tube or catheter 10 will be reduced. When rotation is reversed the size of the annular passage will be progressively increased depending on the setting of the sleeve on the extension until eventually the tube or catheter 10 is in its unstretched condition permitting maximum flow of liquid through the tube.

With such a device as this the size of the internal flow passage or lumen of the tube or catheter 10 will be consistent at all times because the tube or catheter will always retain its natural round section so that no undue stress or strain will be exerted on the material of the tube or catheter.

It will be appreciated that it is not essential to use screw means to move the stretcher bobbins apart. A lever mechanism or cam mechanism could alternatively be used.

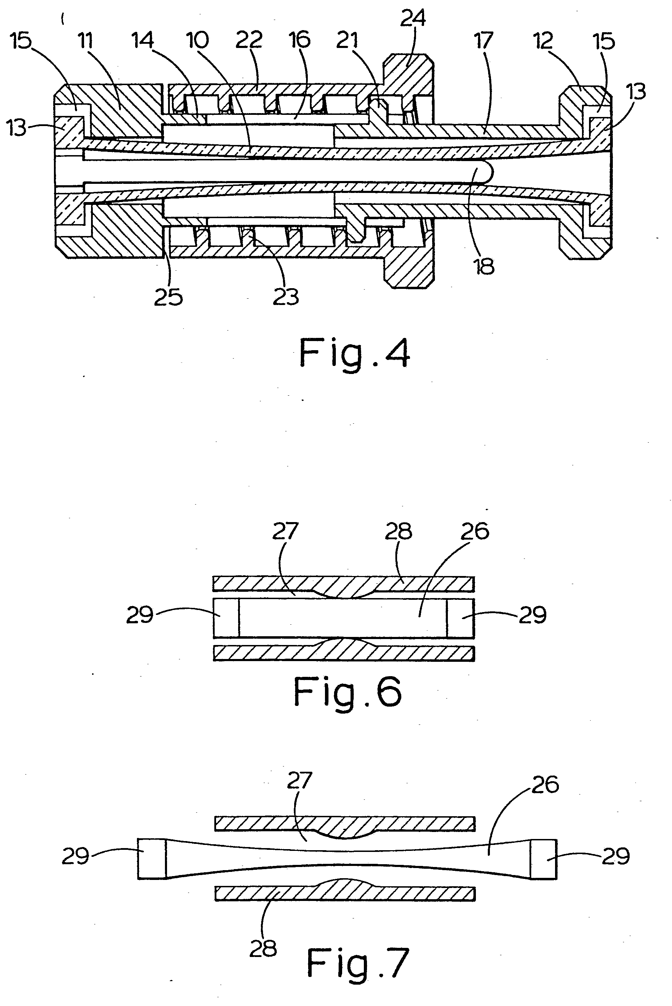

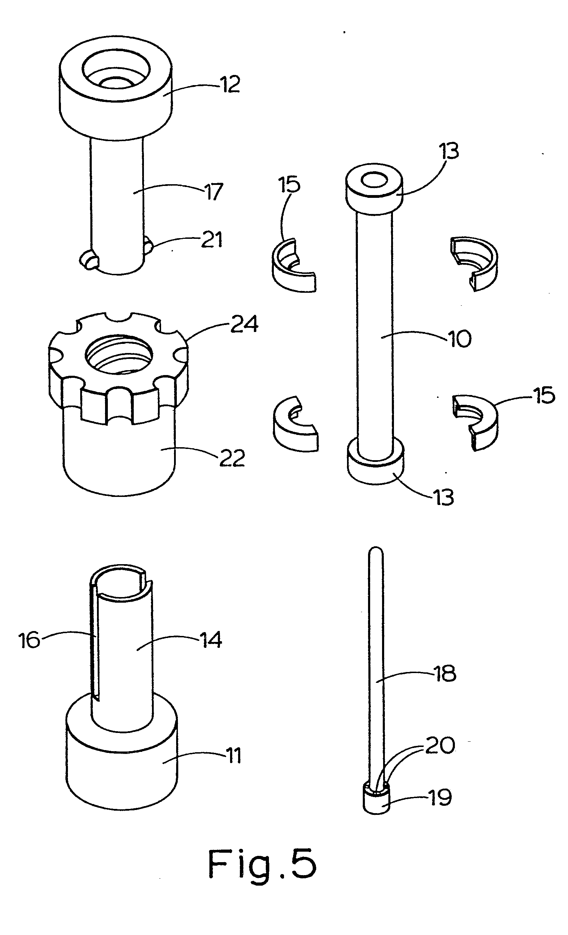

In a modification of the device schematically illustrated in Figures 6 and 7, stretcher bobbins 29 are arranged to stretch or unstretch a stretchable core 26 fitted in a lumen 27 of a tube or catheter 28 which is not stretched but which always maintains its normal condition. As the core 26 is stretched, the size of the annular passage between the core 26 and the inside of the tube 28 will be varied to adjust the flow of liquid or other fluid through the device.

0045668

- 7 -

<u>CLAIMS:</u>

1.    A flow control device comprising a tube, a core
arranged inside the tube, the outside of the core being
spaced from the inside of the tube thereby to define a
passage through which fluid can flow, at least one of
the tube and core being longitudinally stretchable thereby
to vary the size of the passage.

2.    A flow control device comprising a tube which is
stretchable longitudinally of its axis, a core within
the tube, the outside of the core being spaced from the
inside of the tube when the tube is in its unstretched
condition, stretcher members spaced from each other on the
tube or catheter and fixed thereon and means for forcing
apart the stretcher members thereby to stretch the tube.

3.    A flow control device comprising a tube, a core
within the tube, the said core being stretchable longitudinally
of its axis and having its outside spaced from the inside
of the tube when the core is in its unstretched condition,
stretcher members spaced from each other on the core and
fixed thereon, and means for forcing apart the stretcher
members thereby to stretch the core.

4.    A flow control device as claimed in either of
claims 1 or 2, wherein the tube has a pair of spaced
external flanges, a pair of stretcher bobbins are a
sliding fit on the outside of the tube between the
flanges and a screw connection is provided between the
two stretcher bobbins and is operable to move the bobbins
apart so that they bear on the flanges and stretch the
tube.

5.      A flow control device as claimed in claim 4,
wherein one of the stretcher bobbins is a female bobbin
and has an extension with a longitudinal slot therein,
the other stretcher bobbin is a male bobbin and has an
extension which is a sliding fit on the extension of
the female bobbin and has a screw thread projection
located in the slot so that the two stretcher bobbins
cannot rotate with respect to one another and a sleeve
is located on the outside of the female bobbin and has
an internal screw thread in which the screw thread
projection of the male stretcher bobbin is located whereby
rotation of the sleeve will move the two stretcher bobbins
longitudinally along the tube so that the tube will be
stretched or unstretched.

6.      A flow control device as claimed in claims 4 or 5,
wherein the core is secured to the tube by means of a
flange located in the tube and having apertures through
which fluid can flow.

7.      A flow control device substantially as described
with reference to the accompanying drawings.

Fig.1

Fig.2

Fig.3

Fig.4

Fig.6

Fig.7

Fig.5

0045668

European Patent Office

**EUROPEAN SEARCH REPORT**

Application number

EP 81 30 3599

## DOCUMENTS CONSIDERED TO BE RELEVANT

| Category | Citation of document with indication, where appropriate, of relevant passages | Relevant to claim |
|---|---|---|
| PEX | FR - A - 2 456 525 (KONINKLIJKE EMBALLAGE)<br>   * Figures 13,14; page 1, lines 21-31; page 8, lines 6-12; page 9, lines 20-25 *<br><br>-- | 1,2 |
| PX | US - A - 4 275 765 (DUGAS)<br>   * Figures 1,2,3; column 1, lines 28-32; column 1, line 67 - column 2, line 16 *<br><br>-- | 1,3 |
| X | GB - A - 1 117 940 (BROWN)<br>   * Figure 1; page 1, lines 57-73 *<br><br>-- | 1,3 |
| | FR - A - 554 511 (FEHR)<br>   * Figures 3, 4; page 2, lines 6-13 *<br><br>-- | 1 |
| A | US - A - 3 717 174 (DEWALL)<br><br>---- | |

**CLASSIFICATION OF THE APPLICATION (Int. Cl.³)**

A 61 M 5/14
F 16 K 7/08

**TECHNICAL FIELDS SEARCHED (Int. Cl.³)**

A 61 M
F 16 K

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family. corresponding document

The present search report has been drawn up for all claims

| Place of search | Date of completion of the search | Examiner |
|---|---|---|
| The Hague | 21-10-1981 | DURAND-SMET |

EPO Form 1503.1   06.78